# EUROPEAN PATENT SPECIFICATION

(11) **EP 2 968 855 B1**
(45) Date of publication and mention of the grant of the patent: **30.12.2020**
(21) Application number: 14780254.0
(22) Date of filing: 12.03.2014
(51) Int. Cl.: A61M 25/09, A61B 18/00, A61B 5/00, A61B 8/12

(54) **MULTICONDUCTOR GUIDEWIRE WITH CHORDAL SURFACE**
MEHRLEITER-FÜHRUNGSDRAHT MIT SEHNENOBERFLÄCHE
FIL GUIDE MULTICONDUCTEUR AVEC SURFACE CHORDALE

(30) Priority: 12.03.2013 US 201361777260 P
(43) Date of publication of application: 20.01.2016
(73) Proprietor: Lake Region Manufacturing, Inc. d/b/a Lake Region Medical, Chaska, MN 55318 (US)
(72) Inventor: LUPTON, Henry, W., Oranmore Galway (IE)
(74) Representative: J A Kemp LLP
(86) International application number: PCT/US2014/023935
(87) International publication number: WO 2014/164962

(56) References cited:
- WO-A1-02/39079
- WO-A1-2013/028737
- US-A- 5 938 624
- US-A1- 2004 111 139
- US-A1- 2006 052 850
- US-A1- 2007 032 744
- US-A1- 2010 273 358

## Description

This invention relates to medical guidewires having multiple conductive pathways, primarily but not exclusively electrically conductive pathways, extending from substantially their distal ends to substantially their proximal ends.

There is a need in the medical industry for a steerable, torquable (i.e., can be rotated with essentially 1:1 rotational fidelity), pushable, and flexible device capable of navigating complex vascular pathways. Further there is a perceived desire in the medical industry that a device which is capable of navigating complex vascular anatomy also have the capability of providing multiple conductive pathways from about the proximal end of the device to approximately its distal end. These multiple conductive pathways can be used for, e.g., pacing, mapping, sensing, defibrillating and to generally monitor or to treat electrical or electrophysical phenomena within the body from outside the body.

One approach that has been used to provide a multi-conductive pathway is to insert multiple insulated cables or wires inside a hollow tube, e.g., a segment of hypotube. An example of this prior art approach is found in U.S. 6,908,442 and, US-A1-2010/0273358 to von Malmborg et al. "Bending Resistant Male Connector for a Guidewire". At column 4, line 60, a cross- section of a preferred embodiment of Malmborg et al.'s male connector is described and shown at FIG. 5. As disclosed, a core wire having a homogeneous D-shaped cross section constitutes a central part of the connector. Three conductors are positioned on the flat part of the D-shaped core wire, and a cylindrical conductive member surrounds the von Malmborg et al. conductors and the D-shaped core wire. The rest of the space inside the cylindrical conductive member of von Malmborg et al. is filled with insulating material. A cavity is defined by the cylindrical conductive member and the flat part of the D-shaped core wire in which the conductors are disposed.

The insulated-cables-within-a-hypotube approach of Malmborg et al. (and others) has a significant drawback in that a tube does not easily perform the functions of steerability and rotatability so as to permit efficient, familiar (e.g., to a physician) guidewire-like navigation of complex vasculature. A tube also increases overall guidewire diameter. Any cavity filled with insulative material will probably increase overall device diameter which, for medical reasons, is generally to be avoided.

Another approach has been simply to wrap one or more insulted wires about the outside of a solid core guidewire or guidewire core. The expedient of wrapping the insulated conducting cables or wires around a solid core, while providing better vascular navigation capabilities means the overall diameter of the core structure has be reduced to provide lumen or intravascular space to accommodate the cables and core structure in the limited lumen or sectional area available. Reducing core wire or core structure diameter has the drawback or reducing corewire stiffness and performance.

WO-A1-2013/028737 on which the pre-characterizing portion of claim 1 is based discloses a guidewire with a core wire having a helical groove cut in it to accommodate electrical conductors. WO-A1-02/39079 also discloses a guidewire having a core wire with channels in it for housing electrical conductors.

According to the present invention there is provided a guidewire as defined by claim 1.

This invention is a multipolar or multi-conductor guidewire which provides multiple proximal to distal conductive pathways in the absence of a tube. In a preferred practice of this invention, the conductive pathway or pathways are substantially longitudinally parallel to each other running linearly along substantially the majority if not the entire length of the guidewire body.

In practice of this invention multiple contacts on the distal and proximal ends of the guidewire can be created, the guidewire itself providing the properties to permit intravascular placement of those electrical contacts as appropriate to the electromedical phenomena being monitored, altered, or created. Monitoring, sensing, pacing (stimulation), defibrillating, mapping, ablation, and numerous other procedures whereby physical or electrical phenomena are affected or determined within the body will be suggested to one skilled in this art by this disclosure.

There are numerous possible applications of the present approach to the creation of a guidewire assembly having multiple conductive pathways. "Conductive pathways" as that term is used here means pathways having the material characteristics to conduct electricity, light, fluids (possibly by osmosis or other means), heat, vibration, sound waves, e.g., ultrasound, longitudinal movement or motion and rotational movement or motion. One skilled in this art will appreciate that the structure herein described will have many potential applications in the medical field where an intravascular or endovascular procedure is to be accomplished from outside a patient (exovascularly).

Briefly, in one aspect, the present invention is a guidewire having a solid core wire defining a chordal or substantially flat surface or segment. A chord of a circle is a generally straight line connecting two points on the circumference of the circle. The reference to circle here is the outer surface of the guidewire core wire structure when viewed in transverse section. Thus, in one instance, the sectional view of a guidewire core wire of the present invention with its chordal or secant surface could be described as a "D" surface. The flat surface (which is not required to be flat and is thusly sometimes referred to as "faceted" to permit curved, undulating, radiussed or other profile surfaces) may traverse all or part of the guidewire structure or assembly from its distal end to its proximal end in essentially a straight line. Alternatively, the "flat" or faceted surface can define an open helix, with the surface itself defining a number of partial or complete 360 ° "turns" from distal to proximal end of the structure. Disposed upon and supported by the flat surface are one or more conductors, usually electrical conductors. The conductors generally will have electrically insulative coatings disposed therein.

The present invention contemplates the possibility that more than one chordal surface may be created in the guidewire body. Further, the chordal surfaces do no necessarily have to be located so that their edges are next to one another. So, for example, a two chordal surface or two-faceted version of this invention could have the flat surfaces on opposite sides of the guidewire body with a remaining circular segment connecting the edges thereof. For this aspect of the invention, a solid core wire or core shaft is ground or otherwise treated to provide the guidewire body facet or flat disclosed herein.

A chordal guidewire surface of this invention is created by removing a portion of the generally circular-in-section guidewire structure, e.g., a solid core wire, by grinding, cutting, or laser treatment. Such a surface could also be created by drawing a wire structure in a formable or plastic state through a die having the requisite orifice configuration e.g., a "D"-shaped opening. Numerous other techniques that create the required linear or helical chordal surface will be apparent to one skilled in this art in light of this disclosure.

It will be appreciated that removing a portion of a guidewire body or, e.g., its core wire, to create a facet or "flat" has at least some tendency to change the handling characteristics of the remaining structure. While minor changes or diminution of the guidewires handling characteristics are contemplated, no significant guidewire handling characteristics are intended to be changed. Thus, the removal of the guidewire body to create the flat(s) or facet(s) described herein is to be limited by the intention not to materially change the steerability, rotatability and pushability of the remaining guidewire structure.

In one approach, a flat surface is created on a guidewire core wire or shaft. Generally speaking, the core wire, in this approach, will have a flat or flats that traverse the majority of its length. A number of lengths of conductors or fibers or tubes can be laid, i.e., axially placed or disposed, along the flat or chordal surface so created. These axially placed conductors can be retained on the flat(s) using shrink tube, adhesive or other typical means of bonding. This approach overcomes the shortcomings of placing the wire in hypodermic tubing which have poor mechanical handling characteristics. It also allows the distal end to be profiled in the normal guidewire fashion providing a region of reduced stiffness matching, a given anatomy, and the accompanying diagnostic or therapeutic device. The axial strands can be used for a wide variety of purposes such as conducting electricity, light, sound, fluids, etc. The advantage of creating the flat on which to deploy the conductor is to maximize the cross section of the remaining guidewire core material (i.e., to minimize the loss of corewire body) to retain, as much as possible, the guidewire's handling characteristics.

For some applications, the longitudinal strands or conductors may need to be protected from external influences. This may be basic mechanical protection or it may be a shielding of external electromagnetic radiation or indeed to contain signals or light within the guidewire. Screening may be provided, for example, by using a helical or wound coil of wire. Lower profiles may be achieved using flat wire coils. The length of coil used would be held in place, for example, by creation of an interference fit between the inner lumen of the coil and the outside diameter of the core/conductor assembly. It is applied over the core-strand assembly by partially unwrapping the coil by a sufficient amount to increase the diameter of its lumen to allow a loose fit, inserting the chordal guidewire/conductor assembly into the partially unwound coil and releasing the unwrapped coil causing it to bind down on the core. This binding is essential for the transmission of torque down the length of the entire assembly, a critical component of guidewire handling characteristics or "feel" as it is sometimes referred to.

### DETAILED DESCRIPTION OF THE INVENTION

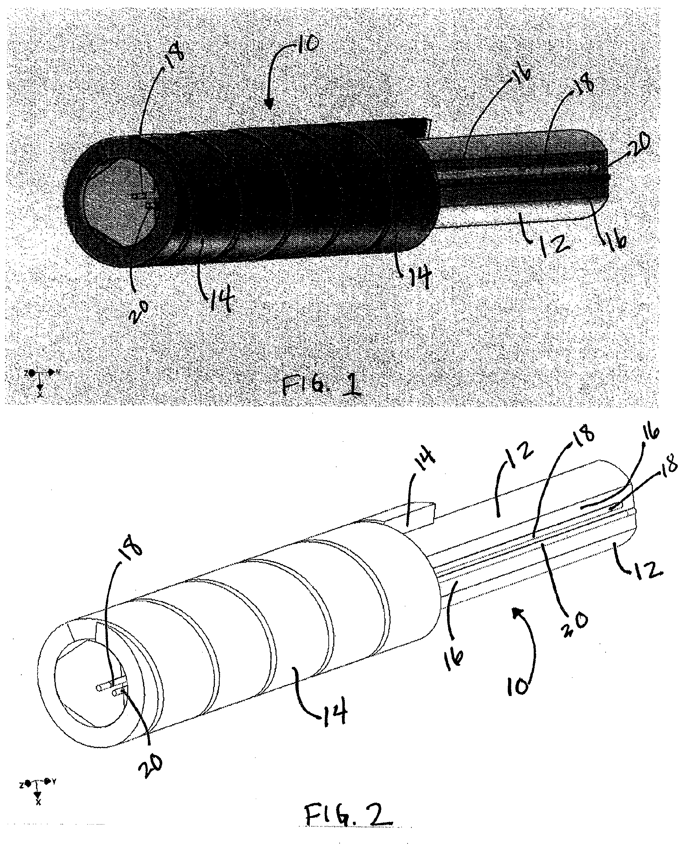

Thus there is shown in the attached Figs. 1 and 2, an exemplary embodiment of the present invention. It is to be appreciated that while the embodiment of the Figs is shown to have one chordal surface or "flat", multiple chordal surfaces are contemplated by the present invention. The exact number of such surfaces will be determined by the extent to which the slight change in guidewire handling characteristics is medically acceptable as each chordal surface or facet is created by partial removal of the guidewire core wire or madril body. It is, of course, not necessary that a chordal surface core wire of this invention be created by removal of core wire material extrusion. Processes e.g., through a die, could accomplish the same result.

Guidewire 10 is an elongate structure comprising a core wire or core element 12 and, in this embodiment a flattened coil 14. Flattened coil 14 is used to create a lower guidewire cross-sectional profile. A circular wire coil (or other coil profile, e.g., oblate or prolate, none of which are shown) also can be used. Coil 14 has been partially cut away to illustrate its relationship with core element 12. Coils comprising more than one material e.g., a stainless steel coil having a radiopaque distal segment, and coaxial coil construction are contemplated. Core element 12 has, in this embodiment, a single chordal surface, flat or facet 16 on which there is disposed two conductors 18, 20. Conductors 18, 20 also are partially broken away for purposes of illustration, it being understood that additional guidewire structure extends to both the right and to the left in the Figures.

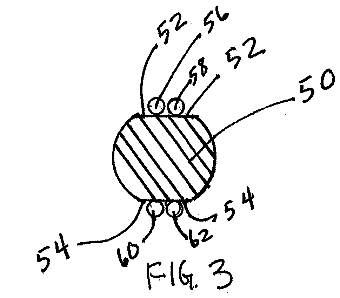

Fig. 3 illustrates in section an embodiment of the present invention in which core element 50, e.g., a guidewire core wire has two facets or chordal surfaces 52, 54. Electrical conductors 56,58, 60 and 62 are shown in section in Fig. 3. Conductors 56, 58, 60 and 62 would be held upon their respective chordal surfaces 52, 54 by any of the means discussed above (also not shown). Conductors 56, 68 and 60-62 would in the normal course be appropriately coated or insulated depending upon the diagnostic or therapeutic application for which the conductive pathways are to be used.

## Claims

1. A guidewire (10) having a proximal segment and a distal segment, the guidewire (10) comprising:
an elongate, solid core wire (12), the core wire (12) having multiple insulated conductors (18, 20) disposed thereon;
**characterized in that** the core wire (12) has a chordal core wire surface (16) on which the multiple insulated conductors (18, 20) are disposed, the chordal core wire surface (16) running at least part of the length of the core wire (12) to the distal segment of the guidewire (10).

2. A guidewire (10) according to claim 1 wherein the guidewire (10) further includes a coil (14) disposed around the core wire (12) and the conductors (18, 20).

3. A guidewire (10) according to claim 1, wherein said chordal core wire surface (16) runs substantially the entire length of the core wire (12) from the proximal segment to the distal segment of the guidewire (10), and has a coil (14) disposed thereon, the coil (14) being disposed around at least the core wire distal segment and being attached to the core wire (12) at its extreme distal end.

4. A guidewire (10) according to claim 2 or claim 3 wherein the coil (14) is a flat wire coil.

5. A guidewire (10) according to claim 2 or claim 3 wherein the coil (14) wraps around substantially the entire length of the core wire (12).

6. A guidewire (10) according to any preceding claim wherein the chordal surface (16) is substantially flat.

7. A guidewire (10) according to any one or claims 1 to 5, wherein the chordal surface (16) is curved, radiussed. or helical.

8. A guidewire (10) according to any one of claims 1 to 5 wherein the chordal surface is helical and runs between the distal end and proximal end of the core wire (12).

9. A guidewire (10) according to any preceding claim wherein the core wire (12) defines multiple chordal surfaces (10).

10. A guidewire (10) according to any preceding claim wherein the conductors (18, 20) are retained upon the chordal surface (16) of the core wire (12) by a shrink tube.

11. A guidewire (10) according to any one of claims 1 to 9 wherein the conductors (18, 20) are adhesively bound to the core wire chordal surface (16).

12. A guidewire (10) according to any preceding claim wherein the multiple insulated conductors (18, 20) themselves comprise flattened wire.

13. A guidewire (10) according to any preceding claim wherein the core wire (12) defines two core wire chordal surfaces (52, 54).

14. A guidewire (10) according to any preceding claim wherein the core wire (12) defines three core wire chordal surfaces (16).

15. A guidewire (10) according to claim 14 wherein at least two insulated conductors (18, 20) are disposed on each of the three core wire chordal surfaces (16).

## Patentansprüche

1. Führungsdraht (10) mit einem proximalen Segment und einem distalen Segment, wobei der Führungsdraht (10) Folgendes umfasst:
einen länglichen festen Kerndraht (12), wobei der Kerndraht (12) mehrere isolierte Leiter (18, 20) aufweist, die darauf angeordnet sind;
**dadurch gekennzeichnet, dass** der Kerndraht (12) eine Sehnenkerndrahtoberfläche (16) aufweist, auf der die mehreren isolierten Leiter (18, 20) angeordnet sind, wobei die Sehnenkerndrahtoberfläche (16) mindestens einen Teil der Länge des Kerndrahtes (12) zum distalen Segment des Führungsdrahtes (10) verläuft.

2. Führungsdraht (10) nach Anspruch 1, wobei der Führungsdraht (10) ferner eine Spule (14) umfasst, die um den Kerndraht (12) und die Leiter (18, 20) angeordnet ist.

3. Führungsdraht (10) nach Anspruch 1, wobei die Sehnenkerndrahtoberfläche (16) im Wesentlichen über die gesamte Länge des Kerndrahtes (12) vom proximalen Segment zum distalen Segment des Führungsdrahtes (10) verläuft, und eine darauf angeordnete Spule (14) aufweist, wobei die Spule (14) mindestens um das distale Segment des Kerndrahtes angeordnet und an seinem äußersten distalen Ende an dem Kerndraht (12) angebracht ist.

4. Führungsdraht (10) nach Anspruch 2 oder Anspruch 3, wobei die Spule (14) eine flache Drahtspule ist.

5. Führungsdraht (10) nach Anspruch 2 oder Anspruch 3, wobei die Spule (14) im Wesentlichen die gesamte Länge des Kerndrahtes (12) umwickelt.

6. Führungsdraht (10) nach einem der vorhergehenden Ansprüche, wobei die Sehnenfläche (16) im wesentlichen flach ist.

7. Führungsdraht (10) nach einem der Ansprüche 1 bis 5, wobei die Sehnenfläche (16) gekrümmt, strahlend oder spiralförmig ist.

8. Führungsdraht (10) nach einem der Ansprüche 1 bis 5, wobei die Sehnenoberfläche spiralförmig ist und zwischen dem distalen Ende und dem proximalen Ende des Kerndrahtes (12) verläuft.

9. Führungsdraht (10) nach einem der vorhergehenden Ansprüche, wobei der Kerndraht (12) mehrere Sehnenoberflächen definiert.

10. Führungsdraht (10) nach einem der vorhergehenden Ansprüche, wobei die Leiter (18, 20) durch ein Schrumpfrohr auf der Sehnenoberfläche (16) des Kerndrahtes (12) gehalten werden.

11. Führungsdraht (10) nach einem der Ansprüche 1 bis 9, wobei die Leiter (18, 20) an die Sehnenoberfläche (16) des Kerndrahtes geklebt sind.

12. Führungsdraht (10) nach einem der vorhergehenden Ansprüche, wobei die mehreren isolierten Leiter (18, 20) selbst abgeflachten Draht umfassen.

13. Führungsdraht (10) nach einem der vorhergehenden Ansprüche, wobei der Kerndraht (12) zwei Kerndrahtsehnenflächen (52, 54) definiert.

14. Führungsdraht (10) nach einem der vorhergehenden Ansprüche, wobei der Kerndraht (12) drei Kerndrahtsehnenflächen (16) definiert.

15. Führungsdraht (10) nach Anspruch 14, wobei mindestens zwei isolierte Leiter (18, 20) auf jeder der drei Kerndrahtsehnenflächen (16) angeordnet sind.

## Revendications

1. Fil de guidage (10) ayant un segment proximal et un segment distal, le fil de guidage (10) comprenant :
un fil central allongé et solide (12), le fil central (12) ayant plusieurs conducteurs isolés (18, 20) disposés dessus ;
**caractérisé en ce que** le fil central (12) a une surface chordale de fil central (16) sur laquelle les multiples conducteurs isolés (18, 20) sont disposés, la surface chordale de fil central (16) s'étendant sur au moins une partie de la longueur du fil central (12) au segment distal du fil de guidage (10).

2. Fil de guidage (10) selon la revendication 1, dans lequel le fil de guidage (10) comprend en outre une bobine (14) disposée autour du fil central (12) et des conducteurs (18, 20).

3. Fil de guidage (10) selon la revendication 1, dans lequel ladite surface chordale de fil central (16) s'étend sensiblement sur toute la longueur du fil central (12) à partir du segment proximal au segment distal du fil de guidage (10), et a une bobine (14) disposée dessus, la bobine (14) étant disposée autour d'au moins le segment distal du fil central et étant fixée au fil central (12) à son extrémité distale.

4. Fil de guidage (10) selon la revendication 2 ou 3, dans lequel la bobine (14) est une bobine de fil plat.

5. Fil de guidage (10) selon la revendication 2 ou 3, dans lequel la bobine (14) s'enroule sensiblement sur toute la longueur du fil central (12).

6. Fil de guidage (10) selon l'une quelconque des revendications précédentes, dans lequel la surface chordale (16) est sensiblement plate.

7. Fil de guidage (10) selon l'une quelconque des revendications 1 à 5, dans lequel la surface chordale (16) est courbe, arrondie ou hélicoïdale.

8. Fil de guidage (10) selon l'une quelconque des revendications 1 à 5, dans lequel la surface chordale est hélicoïdale et s'étend entre l'extrémité distale et l'extrémité proximale du fil central (12).

9. Fil de guidage (10) selon l'une quelconque des revendications précédentes, dans lequel le fil central (12) définit de multiples surfaces chordales (10).

10. Fil de guidage (10) selon l'une quelconque des revendications précédentes, dans lequel les conducteurs (18, 20) sont retenus sur la surface chordale (16) du fil central (12) par une gaine rétractable.

11. Fil de guidage (10) selon l'une quelconque des revendications 1 à 9, dans lequel les conducteurs (18, 20) sont liés de manière adhésive à la surface chordale de fil central (16).

12. Fil de guidage (10) selon l'une quelconque des revendications précédentes, dans lequel les multiples conducteurs isolés (18, 20) comprennent un fil aplati.

13. Fil de guidage (10) selon l'une quelconque des revendications précédentes, dans lequel le fil central (12) définit deux surfaces chordales de fil central (52, 54).

14. Fil de guidage (10) selon l'une quelconque des revendications précédentes, dans lequel le fil d'âme (12) définit trois surfaces chordales de fil central (16).

15. Fil de guidage (10) selon la revendication 14, dans lequel au moins deux conducteurs isolés (18, 20) sont disposés sur chacune des trois surfaces chordales de fil central (16).
